# EUROPEAN PATENT SPECIFICATION

(11) **EP 4 090 402 B1**
(45) Date of publication and mention of the grant of the patent: **02.04.2025**
(21) Application number: 21705693.6
(22) Date of filing: 12.01.2021
(51) Int. Cl.: A61M 5/00, A61M 5/32, A61M 5/31, A61M 5/34, A61M 5/28

(54) **PRIMARY CONTAINER ASSEMBLY WITH INTEGRATED FLUID PATH**
PRIMÄRBEHÄLTERANORDNUNG MIT INTEGRIERTEM FLUIDWEG
ENSEMBLE RÉCIPIENT PRIMAIRE À TRAJET DE FLUIDE INTÉGRÉ

(30) Priority: 16.01.2020 US 202062961933 P
(43) Date of publication of application: 23.11.2022
(73) Proprietor: West Pharmaceutical Services, Inc., Exton, PA 19341 (US)
(72) Inventor: DAVIS, Tommy Gene, Athens, TX 75751 (US); VORA, Rohit, Exton, PA 19341 (US); HEZKIAHU, Ran, 4668346 Herzliya (IL)
(74) Representative: Finlayson, Scott Henry
(86) International application number: PCT/US2021/013017
(87) International publication number: WO 2021/146154

(56) References cited:
- WO-A1-2018/226515
- KR-A- 20090 120 043

## Description

### CROSS-REFERENCE TO RELATED APPLICATIONS

This application claims the benefit of U.S. Provisional Patent App. No. 62/961,933, filed Jan. 16, 2020.

### TECHNICAL FIELD

This application is directed to a primary container assembly for storing and dispensing a drug. Specifically, this application relates to a primary container assembly for ensuring sterility of a fluid path from manufacturing, through introduction of the drug in the primary container assembly, and to the end user.

### BACKGROUND

In order to dispense a drug into the subcutaneous tissue, intramuscular tissue, or veins of a patient, a fluid path is required to allow the drug to flow out of a primary container storing the drug and through a needle. As a result, an interface is required between the primary container, fluid path, and needle. To form such a connection, conventional primary containers can have an adaptive end (septum, Luer lock) or other interfacing features. However, when interfacing the primary container with the fluid path, needle, or other components of a dispensing system, sterility of the primary container assembly can be compromised. For example, when interfacing a primary container, fluid path, and needle, the connection point between each of these components can have compromised sterility during or after connection, thus leading to a loss of sterility of the system. This can necessitate swabbing of various fittings with alcohol wipes prior to connection to mitigate or minimize risk, leading to greater process complexity. Additionally, such interfacing efforts often require an arrangement of several unique and specially designed interface features that further increase the components through which sterility can be compromised, as well as increase costs and complexity associated with assembling a dispensing device with the goal of ensuring end-to-end sterility.

As a result, there is a need for a primary container assembly having decreased complexity that ensures end-to-end sterility in a drug loading and dispensing process.

WO2018/226515 describes a syringe assembly for a drug delivery device. The syringe assembly includes a syringe barrel having a proximal end, a distal end, and a longitudinal axis. A needle assembly is operatively coupled to the syringe barrel and includes a needle hub and a needle attached to the needle hub. A flexible connection is disposed between the syringe barrel and the needle hub and forms a fluid pathway between the syringe barrel and the needle. The flexible connection enables the needle assembly to be moveable from a filling position, in which a longitudinal axis of the needle assembly is parallel to a longitudinal axis of the syringe barrel, to an assembled position, in which the longitudinal axis of the needle assembly is not parallel to the longitudinal axis of the syringe barrel.

### SUMMARY

The invention to which this European patent relates is defined in the appended claims.

An example described herein is a primary container assembly comprising a primary container defining a body having a proximal end defining an opening configured to receive a plunger, a distal end opposite the proximal end and defining an outlet, and a chamber extending from the proximal end to the distal end that is configured to receive a drug. The primary container assembly further comprises a tube extending from a first end that is integrally attached to the distal end of the primary container to a second end opposite the first end, where the tube defines a channel extending from the first end to the second end. The primary container assembly also comprises a hollow needle configured to penetrate skin of a patient, wherein the hollow needle is integrally attached to the second end of the tube, where the tube is configured to direct the drug from the chamber of the primary container to the needle.

Also described herein is a method for assembling a primary container assembly. The method includes providing a primary container defining a body having a proximal end defining an opening configured to receive a plunger, a distal end opposite the proximal end and defining an outlet, and a chamber extending from the proximal end to the distal end that is configured to receive a drug. The method further includes attaching a first end of a tube to the distal end of the primary container, such that the tube is in fluid communication with the outlet, and attaching a hollow needle to a second end of the tube.

### BRIEF DESCRIPTION OF THE DRAWINGS

The foregoing summary, as well as the following detailed description, will be better understood when read in conjunction with the appended drawings. The drawings show illustrative embodiments of the disclosure. It should be understood, however, that the application is not limited to the precise arrangements and instrumentalities shown.
FIG. 1 is an exploded view of a primary container assembly according to an embodiment of the present disclosure;
FIG. 2 is a side view of the primary container and a tube of the primary container assembly shown in FIG. 1, where the tube is directly attached to the primary container;
FIG. 3 is a side view of the primary container and tube of the primary container assembly shown in FIG. 1, where the tube is attached to the primary container via a needle;
FIG. 4 is a perspective view of the tube of the primary container assembly shown in FIG. 1 in an uncoiled configuration;
FIG. 5A is a perspective view of a needle hub and needle of the primary container assembly according to an embodiment of the present disclosure;
FIG. 5B is a perspective view of a needle hub and needle shield of the primary container assembly according to a further embodiment of the present disclosure;
FIG. 6A is a cross-sectional view of the needle hub and needle shown in FIG. 5A;
FIG. 6B is a cross-sectional view of the needle hub and needle shown in FIG. 5A, with a needle shield attached.
FIG. 7A is a cross-sectional view of the primary container assembly shown in FIG. 1, with a single cup attached to the primary container;
FIG. 7B is a cross-sectional view of the primary container assembly shown in FIG. 1, with two cups attached to the primary container;
FIG. 8 is a schematic view of components of a primary container assembly according to the present disclosure installed within a drug dispensing device;
FIG. 9 is a process flow diagram of a method of assembling a primary container assembly according to an embodiment of the present disclosure;
FIG. 10A is a perspective view of the primary container assembly shown in FIG. 7B in combination with a compatible nest assembly; and
FIG. 10B is a side cross-sectional view of the primary container assembly and nest assembly shown in FIG. 10A, with the primary container assembly supported in a filling position.

### DETAILED DESCRIPTION OF ILLUSTRATIVE EMBODIMENTS

Described herein are primary container assemblies 10, 10' for storing and dispensing a supply of a drug. Certain terminology is used to describe the primary container assemblies 10, 10' in the following description for convenience only and is not limiting. The words "right", "left", "lower," and "upper" designate directions in the drawings to which reference is made. The words "inner" and "outer" refer to directions toward and away from, respectively, the geometric center of the description to describe primary container assemblies 10, 10' and related parts thereof. The words "forward," "rearward," "proximal," and "distal" refer to directions toward or away the proximal or distal ends of body of the component of the primary container assemblies 10, 10' being referred to. The terminology includes the above-listed words, derivatives thereof and words of similar import.

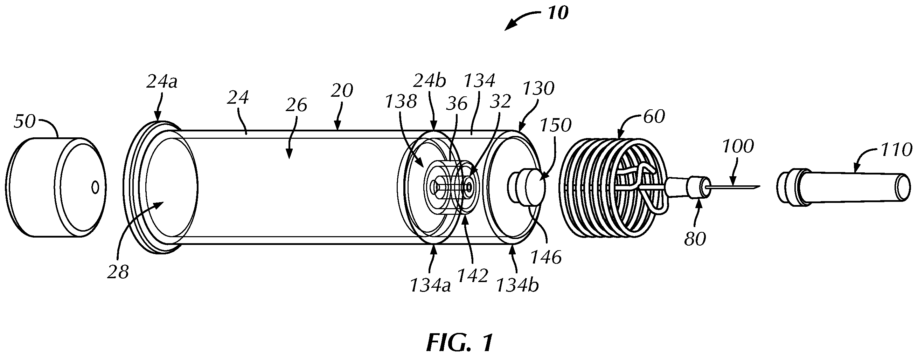

An embodiment of the present disclosure comprises a primary container assembly 10 configured to store and deliver a supply of a drug. The primary container assembly 10 can include a primary container 20, plunger 50, tube 60, needle 72, needle hub 80, needle 100, needle shield 110, cup 130, and/or second cup 160, each of which will be described in detail below. Referring to FIGS. 1-3, the primary container 20 can define a body 24 that extends from a proximal end 24a to a distal end 24b opposite the proximal end 24a, where the body 24 can be comprised of a plastic, such as cyclic olefin or other medical grade plastic, or glass. The body 24 can define a substantially elongate, cylindrical shape, though other shapes are contemplated. In one embodiment, the primary container 20 is configured as a syringe as described in International Patent Application No. PCT/US2015/049588, assigned to West Pharmaceutical Services, Inc. of Exton, PA, though the present disclosure is not intended to be limited to such. The body 24 of the primary container 20 further defines a chamber 26 extending from the proximal end 24a to the distal end 24b, where the chamber 26 is configured to receive and store a supply of a drug, and from which the drug is ultimately dispensed, as will be described below.

The proximal end 24a of the primary container 20 can define a flange to enable easier grasping or ensure proper positioning of the primary container 20 within a dispensing device. The proximal end 24a can also define an opening 28 configured to provide access to the chamber 26. Likewise, the distal end 24b can define an outlet 32 configured to further provide access to the chamber 26. The opening 28 can define a diameter that is larger than a diameter of the outlet 32, such that the opening 28 is configured to receive a plunger 50. In operation, the chamber 26 can be filled from a drug source (not shown) through the opening 28. The plunger 50 can be disposed within the chamber 26 after the chamber 26 has been filled so as to prevent drug from leaking out of the chamber 26 through the opening 28. Further, in operation the plunger 50 can be translated distally through the chamber 26 while sealingly engaging the inner surface of the body 24 so as to force the drug from the chamber 26 through the outlet 32 while maintain a fluid seal with the primary container 20. The plunger 50 can comprise a conventional rubber or plastic plunger, though other embodiments are contemplated. The plunger 50 can be operably attached to an actuation mechanism (not shown) for selectively translating the plunger 50 through the chamber 26, where the actuation mechanism can comprise a motor, spring, pneumatic device, telescoping assembly, or other means capable of causing linear actuation. In one embodiment, the distal end 24b of the primary container 20 comprises a Luer connection, though the distal end 24b can include any structure capable of interfacing with a length of tube 60, which will be described further below.

Referring to FIGS. 1-5B, the tube 60 of the primary container assembly 10 will be described in greater detail. The tube 60 can have a body 64 that extends from a first end 64a to a second end 64b opposite the first end 64a. The body 64 can define a channel 68 extending therethrough from the first end 64a to the second end 64b, such that the body 64 is substantially configured as a hollow tube. The body 64 can be comprised of a medical grade plastic, metal, such as steel or nitinol, etc., though the present disclosure is not intended to be limited to such. In one embodiment, the body 64 is substantially rigid, and as a result defines a fixed shape. In another embodiment, the body 64 is flexible, and as a result can be reconfigured and reshaped by a manufacturer or user as desired. For example, the tube 60 can be coiled during initial packaging and uncoiled during end use. To place the tube 60 in the coiled configuration, the tube 60 can be sequentially wrapped around an extension 36 of the primary container 20 so as to cause at least a portion of the tube 60 to take a coiled configuration comprising a plurality of axially arranged coils. A cup 130 can be placed over the coiled portion of the tube 60 to secure the tube 60 in the coiled configuration, as will be described below. However, in other embodiments the cup 130 can also comprise a portion of the body 24 of the primary container 20 (molded together or as an attachment).

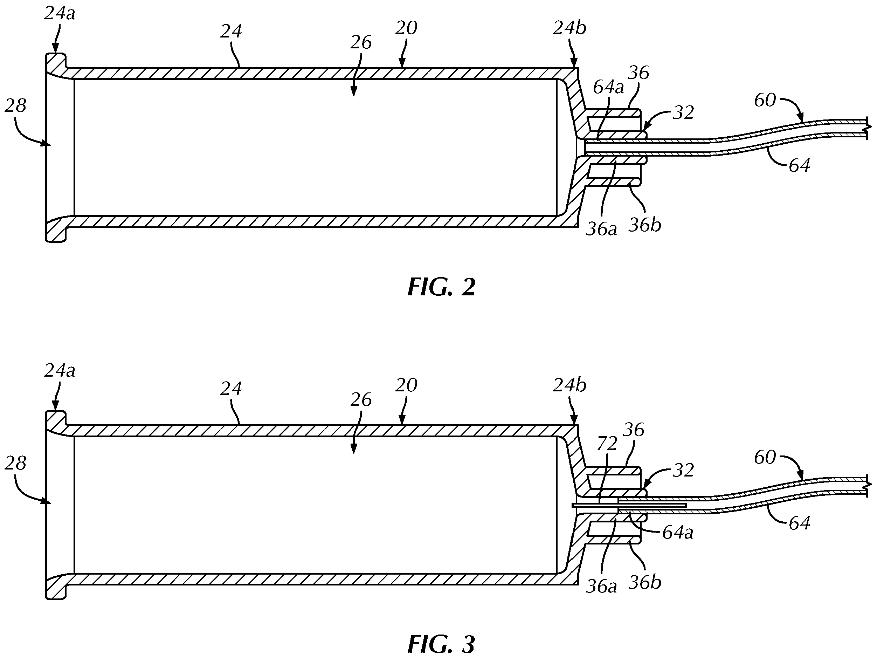

As shown in FIGS. 2 and 3, the first end 64a of the tube 60 can be integrally attached to the distal end 24b of the primary container 20. Specifically, the distal end 24b of the primary container 20 can include an extension 36 extending distally therefrom, where the extension 36 can comprise one or more concentrically arranged hollow cylindrical necks. For example, in the depicted embodiment the extension 36 comprises an inner neck 36a and an outer neck 36b concentrically positioned around the inner neck 36a, though other embodiments are contemplated. The inner neck 36a of the extension 36 can define the portion of the distal end 24b that defines the outlet 32. In one embodiment (FIG. 2), the extension 36 of the primary container 20 can be molded over the first end 64a of the tube 60. In another embodiment, the first end 64a of the tube 60 can be integrally attached to a Luer connection (not shown) of the primary container 20. Optionally, the extension 36 of the primary container 20 can be molded over a portion of a needle 72 (FIG. 3), where the first end 64a of the tube 60 is configured to engage another portion of the needle 72. As a result, the needle 72 can define an interface between the primary container 20 and the tube 60. It is contemplated that the tube 60 can be irremovably attached to the primary container 20. It is also contemplated that the primary container 20 and tube 60 can be formed such that the primary container 20 and tube 60 define a one-piece, monolithic structure, such as through co-injection molding. In another embodiment, an interface component (not shown) can be attached to the first end 64a of the tube 60, where the interface component is configured to snap onto or frictionally engage the distal end 24b of the primary container 20.

The second end 64b of the tube 60 can be integrally attached to a needle hub 80 that defines an interface between a hollow needle 100 and the tube 60. Though depicted as a hollow needle, it is contemplated that in other embodiments the needle 100 can be replaced with a rigid or soft cannula. The needle 100 can comprise an elongate, hollow metal needle, though other configurations are contemplated. For example, the body of the needle 100 can define a 90-degree angle in some embodiments, or any other angle desired. The needle 100 can define a body 104 that extends from a first end 104a to a second end 104b opposite the first end 104a. The second end 104b of the hollow needle 100 can be configured to penetrate skin of a patient, i.e., administer the drug from the chamber 26 of the primary container 20 to the patient. As such, the second end 104b can define a sharp tip. Whereas the primary container 20 is attached to the first end 64a of the tube 60, the first end 104a of the needle 100 is attached to the second end 64b of the tube 60. Due to the inclusion of the tube 60, in this configuration the number of materials used to create the fluid path is minimized, thus eliminating multiple adhesive joints. Further, this design provides the ability to place the needle 100 anywhere on the delivery device without any structural constraints related to the primary container 20.

The primary container assembly 10 can further include a needle hub 80 attached to the needle 100. The needle hub 80 can have a body 82 defining an outer surface 82a, as well as a channel 84 extending therethrough. The second end 64b of the tube 60 is configured to be secured at least partially within the channel 84 of the needle hub 80. Additionally, the hollow needle 100 is configured to be secured at least partially within the channel 84. It is contemplated that in some embodiments, the needle 100 can be at least partially received within the channel 68 of the tube 60.

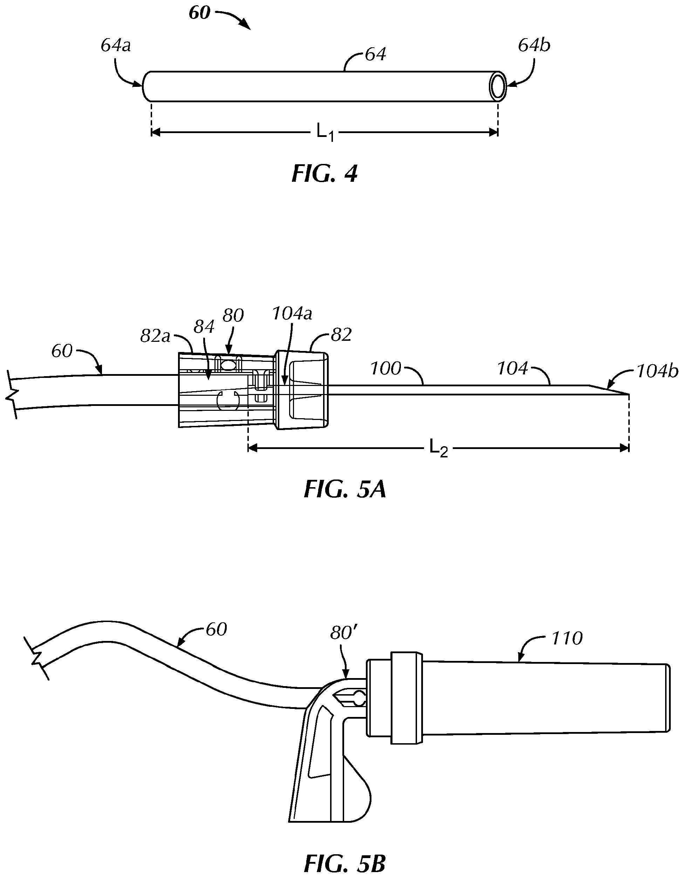

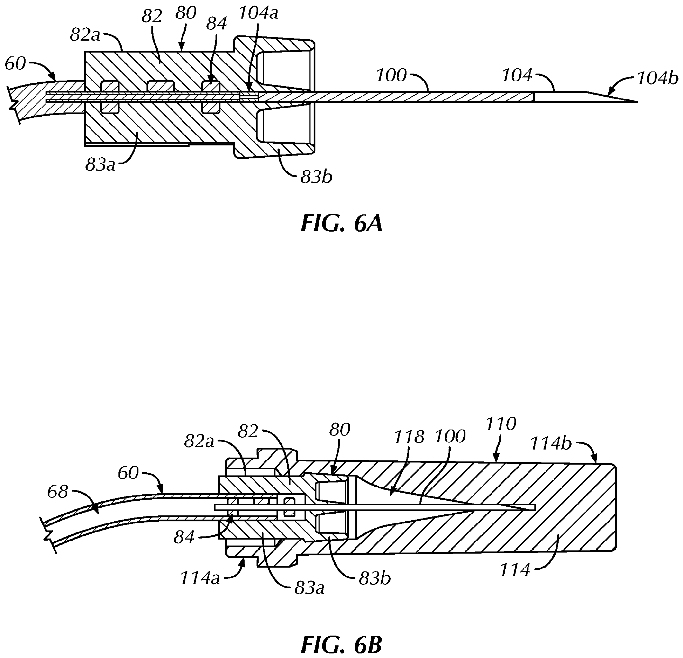

A first embodiment of a needle hub 80 is shown in FIG. 5A, 6A, and 6B, while a second embodiment of a needle hub 80' is shown in FIG. 5B. The body 82 of the needle hub 80 can extend substantially coaxially along a singular axis. Further, a portion of the body 82 can be frustoconical in shape, the function of which will be described below. For example, the body 82 of the needle hub 80 can define a first section 83a at the proximal end of the body 82, and a second section 83b extending distally from the first section 83a. The first section 83a can define a substantially elongate tube, while the second section 83b can have a frustoconical shape. In contrast, the needle hub 80' can have a portion that extends at substantially a 90-degree angle relative to another portion of the needle hub 80'. It is contemplated that the needle hubs 80, 80' can be irremovably attached to the tube 60. The different shapes of the needle hubs 80, 80' can be configured to create particular interferences with primary container assembly components or components of the device within which the primary container assembly 10, 10' is to be received.

As shown in FIG. 4, the tube 60 can define a first length L₁ measured along an axis coaxial with the body 64 of the tube 60 from the first end 64a to the second end 64b. Likewise, the needle 100 can define a second length L₂ measured from its base to its skin-piercing tip, where the first length L₁ is greater than the second length L₂. Optionally, the first length L₁ can be two times, three time, four times, etc. greater than the second length L₂.

As described above, the primary container assembly 10 is configured to cause, under force applied by the plunger 50, the drug to flow from the chamber 26 of the primary container 20, through the tube 60, through the needle hub 80 and needle 100, and into the patient. The configuration of the primary container 20, tube 60, and needle 100 as an integral assembly creates ensured sterility of the drug environment throughout transportation and initial setup of the primary container assembly 10 at the end use site. In conventional primary containers, the primary container must be fluidly attached to other components of a dispensing system through complicated and time intensive processes at the end use site, potentially compromising the sterility of the fluid pathway. The primary container assembly 10 provides a singular fluid path with greatly increased risks for compromised sterility.

To further ensure fluid pathway sterility and prevent unintended injury from contact with the needle 100, the primary container assembly 10 can include a needle shield 110 disposed over at least a portion of the needle 100 and releasably connected to the needle hub 80. Referring to FIG. 6B, the needle shield 110 can have a body 114 that extends from a first end 114a to a second end 114b opposite the first end 114a. It is contemplated that the needle shield 110 can be comprised of a soft or rigid material. For example, the body 114 of the needle shield 110 can be comprised of rubber, though other types of materials are contemplated. The needle shield 110 can define a cavity 118 extending into the body 114 from the first end 114a, where the cavity 118 terminates at a location axially between the first and second ends 114a, 114b. In operation, the cavity 118 can be sized to receive a portion of the needle 100 and at least a portion of the needle hub 80 so as to cover the needle 100, thus ensuring sterility of the needle 100 and preventing injury from human contact with the second end 104b of the needle 100.

When disposed within the cavity 118, a portion of the needle hub 80 can frictionally engage the needle shield 110 so as to secure the needle shield 110 to the needle hub 80. For example, the second section 83b (the frustoconical portion) can frictionally engage the needle hub 80. When the needle hub 80 is inserted into the cavity 118, a progressively increasing diameter of the second section 83b of the needle hub 80 can come into contact with the needle shield 110, thus leading to an eventual frictional engagement with the needle shield 110. Additionally, the cavity 118 can be designed so as to ensure that a predetermined exposure length of the needle 100 is maintained throughout transportation of the needle assembly 10. By maintaining a predetermined length of the needle 100 in contact with the needle shield 110 through transportation, the primary container assembly 10 can ensure sterility of the needle 100 is maintained and the needle 100 does not repeatedly impact the needle shield 100 during transportation, which may otherwise create material fragments.

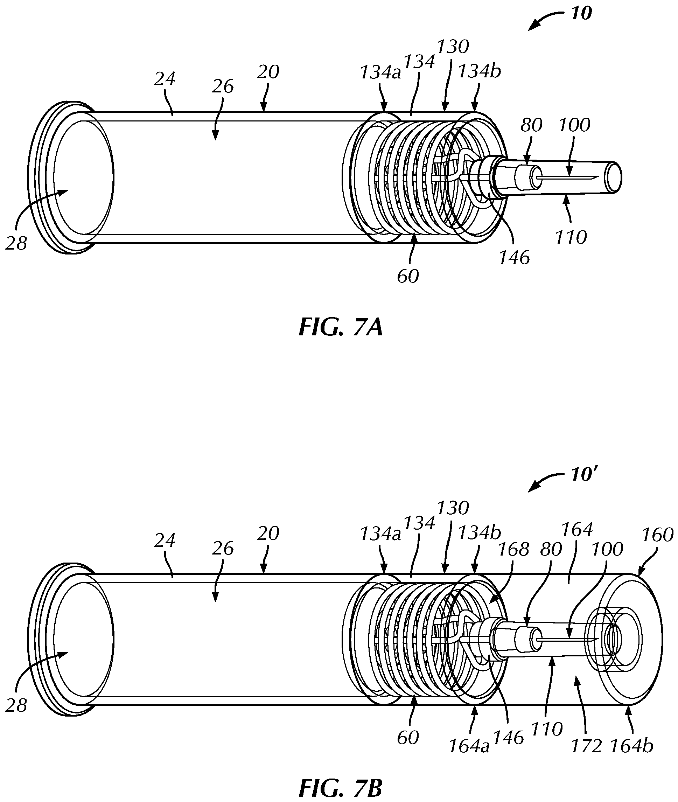

Referring to FIG. 7A, the primary container assembly 10 can further include a cup 130 attached to the distal end 24b of the primary container 20. The cup 130 can have a substantially hollow body 134 that extends from an open proximal end 134a to a substantially closed distal end 134b. Specifically, the proximal end 134a can define an opening 138, and the body 134 defines a cavity 142 extends into the body 134 from the opening 138. The proximal end 134a can be configured to releasably attach to the distal end 24b of the primary container 20. In one embodiment, the cup 130 can be frictionally attached to the primary container 20. However, it is contemplated that the cup 130 can be attached to the primary container 20 through a snap fit, threaded engagement, label applied or shrink-wrapped to the primary container 20 and cup 130, etc. When attached to the primary container 20, the cup 130 can be disposed at least partially over the tube 60 when the tube 60 is in the coiled configuration. As a result, when the tube 60 is placed in the coiled configuration, the tube 60 can be substantially received within the cavity 142 of the cup 130. This can function to keep the tube 60 in the coiled configuration throughout transportation until the primary container assembly 10 is unpackaged for filling or use.

Once the needle shield 110 is attached to the needle hub 80, the cup 130 can be utilized to secure the needle shield 110 to the primary container 20. As shown in FIG. 7A, the cup 130 can include an extension 146 extending from its distal end 134b, where the extension 146 defines a channel 150 extending therethrough. When the cup 130 is placed over the portion of the tube 60 in the coiled configuration and releasably attached to the primary container 20, the needle hub 80 and at least a portion of the tube 60 attached thereto can be fed through the channel 150 of the extension 146, at which point the needle shield 110 can be disposed over the needle hub 80 and the needle 100. The needle shield 110 can further be releasably secure to the cup 130, and in particular the extension 146 of the cup 130. The needle shield 110 can be releasably attached to the cup 130 through a snap fit, threaded engagement, label applied or shrink-wrapped to the needle shield 110 and cup 130, etc.

Once the primary container assembly 10 has reached the final assembly, the components can be unpackaged as follows. First, the needle shield 110 can be detached from the cup 130 and the needle hub 80. Then, the cup 130 can be detached from the primary container 20 and removed from placement over the tube 60 in the coiled configuration. At this point, the user can be free to transition the tube 60 from the coiled configuration to an uncoiled configuration, in which the tube 60 is uncoiled from around the distal end 24b of the primary container, particularly the extension 36 of the primary container 20.

Though the primary container assembly 10 is depicted and described as including the cup 130 for securing the tube 60 in the coiled configuration, it is contemplated that in other embodiments other devices can be utilized for this purpose. For example, it is contemplated that circumferential grooves (for example, spiraling threads) can be defined by the extension 36, where the tube 60 can be coiled around the extension 36 such that the tube 60 is fitted within the grooves to secure the tube 60 to the extension 36. In this or other configurations, the cup 130 may or may not be included.

FIG. 7A depicts a primary container assembly 10 including a single cup 130 utilized to secure the tube 60 in the coiled configuration and the needle shield 110 to the primary container 20. However, as shown in FIG. 7B, another embodiment of a primary container assembly 10' is depicted that includes a second cup 160. The primary container assembly 10 has many similar features as the primary container assembly 10', and such features will be similarly labeled and not described herein for brevity. The second cup 160 can have a body 164 defining an open first end 164a and a substantially closed second end 164b opposite the first end 164a. Specifically, the first end 164a can define an opening 168, and the body 164 defines a cavity 172 extends into the body 164 from the opening 168. The first end 164a can be configured to releasably attach to the distal end 134b of the cup 130. In one embodiment, the second cup 160 can be frictionally attached to the first cup 130. However, it is contemplated that the second cup 160 can be attached to the cup 130 through a snap fit, threaded engagement, label applied or shrink-wrapped to the cup 130 and second cup 160, etc. Alternatively, the cup 130 and second cup 160 can be formed as a monolithic body. In such an embodiment, the cup 130 and second cup 160 can be formed with breakable members at their interface for manual separation of the cup 130 and second cup 160.

When attached to the cup 130, the second cup 160 can be disposed at least partially over the needle shield 110 when the tube 60 is in the coiled configuration. Additionally, the second cup 160 can be disposed at least partially over the needle hub 80 and needle 100 when the needle 100 is received within the needle shield 110. The second cup 160 can function to secure the needle shield 110 during transport of the primary container assembly 10' and offer further protection to the sterility of components of the primary container assembly 10'. Though embodiments of a primary container assembly 10, 10' including cup 130 and/or second cup 160 are shown and described in relation to FIGS. 7A and 7B, it is contemplated that in other embodiments of a primary container assembly, no such cups may be included. One benefit of the embodiment shown in FIG. 7B is the symmetry of the primary container assembly 10' about its central axis, which provides certain benefits during filling, handling, and inspection of the primary container 20. For example, the second cup 160 is configured to interface with a conventional filling machine.

The primary container assemblies 10, 10' can be advantageous in that they define a relatively constant mass about their longitudinal central axis, which allows them to be filled and inspected in conventional ways. For example, the primary container assemblies 10, 10' can be rotated at a high RPM during camera inspection of the drug contents. Such processes would be difficult with a non-symmetric mass around the longitudinal central axis, especially one that allows the needle to be biased away from the central axis line of the primary container.

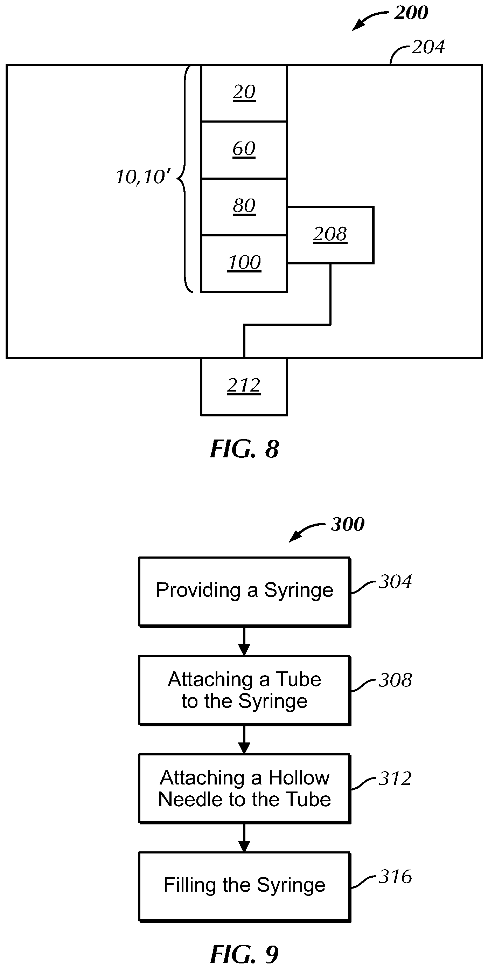

Referring to FIG. 8, a schematic diagram is depicted of a dispensing device 200 configured to dispense a drug. For example, the dispensing device 200 can be a wearable injection device, handheld injection device, or type of device capable of injecting a drug into a patient, though other types of devices are also contemplated. As shown in FIG. 8, the dispensing device 200 can be configured to utilize the primary container assembly 10, 10' as the source for the drug. In one embodiment, the primary container assembly 10, 10' can be manually loaded into the dispensing device 200 by the end user (after removing needle shield 110, cup 130, and/or second cup 160, as described above). Alternatively, the dispensing device 200 can be pre-loaded with components of the primary container assembly 10, 10' by the manufacturer of the dispensing device 200.

The dispensing device 200 can include a body 204 configured to at least partially receive and secure components (such as the primary container 20, plunger 50, tube 60, and needle hub 80) of the primary container assembly 10, 10'. As such, the body 204 can be at least partially hollow. The dispensing device 200 can further include an input 208 configured to engage the needle hub 80 and/or needle 100 when components of the primary container assembly 10, 10' are installed within the body 204 of the dispensing device 200. This engagement can be such that the tube 60 is in fluid communication with the input 208. The dispensing device 200 can also include an output component 212 in fluid communication with the input 208. The output component 212 can be a needle or cannula configured to pierce the skin of a patient, though other output components are contemplated. Alternatively, it is contemplated that the dispensing device 200 can include no output component 212, and rather the needle 100 of the primary container assembly 10, 10' can function as the output component. In operation, when the components of the primary container assembly 10, 10' are received within the dispensing device 200, the needle hub 80 and/or the needle 100 can releasably engage the input 208. For example, the engagement can comprise an interference fit, threaded engagement, snap-fit, etc., though other types of attachment are also contemplated. When the needle hub 80 and/or the needle 100 are engaged with the input 208, the dispensing device 200 can be configured to selectively dispense the drug from the primary container 20, through the tube 60, through the needle hub 80 and needle 100 and into the input 208, from the input 208 to the output component 212, and out of the output component 212 to the patient. A benefit of utilizing the primary container assembly 10, 10' is that the dispensing device 200 does not require sterilization prior to attachment of the primary container assembly 10, 10', as may be the case in other dispensing devices. This is because the fluid path defined by the primary container assembly 10, 10' maintains sterility throughout assembly.

Referring to FIG. 9, a method 300 for assembly of the primary container assembly 10, 10' is depicted. Method 300 can begin with step 304, which includes providing the primary container 20, where the primary container 20 defines a body 24 having a proximal end 24a defining an opening 28 configured to receive a plunger 50, a distal end 24b opposite the proximal end 24a and defining an outlet 32. The primary container 20 also defines a chamber 26 extending from the proximal end 24a to the distal end 24b and is configured to receive a drug. Step 308 can include attaching a first end 64a of the tube 60 to the distal end 24b of the primary container 20, such that the tube 60 is in fluid communication with the outlet 32. Step 308 can comprise molding the distal end 24b of the primary container 20 over the first end 64a of the tube 60. Step 308 can further comprise monolithically forming the tube 60 and the primary container 20. After step 308, in step 312 a hollow needle 100 can be attached to the second end 64b of the tube 60. Then, in step 316, the primary container 20 can be filled with the drug.

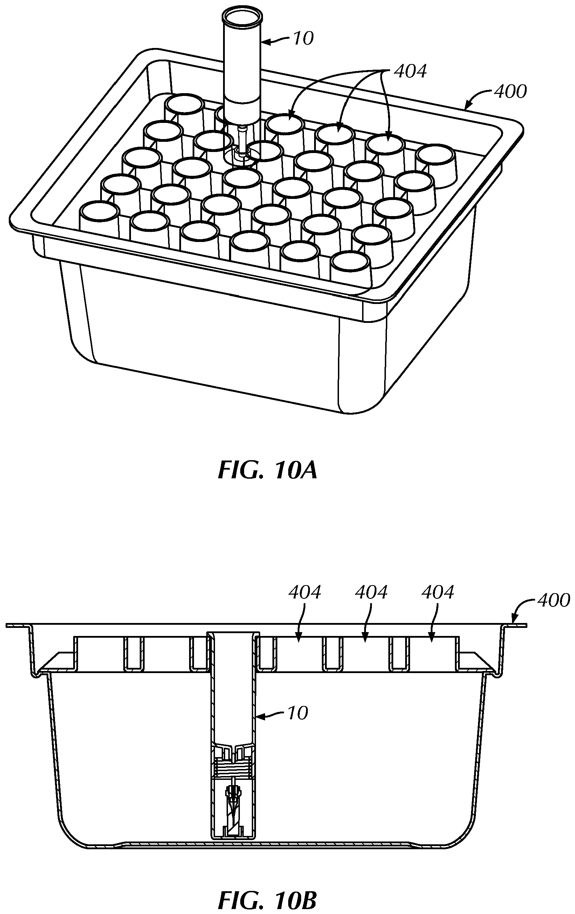

Referring to FIGS. 10A and 10B, the primary container assembly 10, 10' can be configured to interface with a nest assembly 400. Specifically, the nest assembly 400 can define a plurality of apertures 404 configured to receive a respective one of the primary container assemblies 10, 10'. As shown particularly with respect to FIG. 10B, which a primary container assembly 10, 10' is received by the nest assembly 400 in a position where the primary container assembly 10, 10' is oriented for filling with a medicament, none of the tube 60, needle shield 110, cup 130, or other related components interfere with the nest assembly 400 or the orientation of the primary container assembly 10, 10' generally.

While the invention is described herein using a limited number of embodiments, these specific embodiments are not intended to limit the scope of the invention as otherwise described and claimed herein. The precise arrangement of various elements and order of the steps of articles and methods described herein are not to be considered limiting. For instance, although the steps of the methods are described with reference to sequential series of reference signs and progression of the blocks in the figures, the method can be implemented in any particular order as desired.

## Claims

1. A primary container assembly, comprising:
a body (24) having:
a proximal end (24a) defining an opening (28) configured to receive a plunger (50);
a distal end (24b) opposite the proximal end (24a) and defining an outlet (32); and
a chamber (26) extending from the proximal end (24a) to the distal end (24b) that is configured to receive a drug;
a tube (60) extending from a first end (64a) that is integrally attached to the distal end (24b) to a second end (64b) opposite the first end (64a), wherein the tube (60) defines a channel (68) extending from the first end (64a) to the second end (64b);
a hollow needle (100) configured to penetrate skin of a patient, wherein the hollow needle (100) is integrally attached to the second end (64b) of the tube (60); and
wherein the tube (60) is configured to direct the drug from the chamber (26) to the hollow needle (100),
**characterised in that**
the primary container assembly further comprises a cup (130) attached to the distal end (24b) and
wherein the tube (60) is configured to be placed in a coiled configuration, in which a portion of the tube (60) is coiled around a portion of the distal end (24b) and substantially disposed within the cup (130).

2. The primary container assembly of claim 1, comprising a primary container which defines the body (24).

3. The primary container assembly of claim 1 or claim 2, wherein the tube (60) comprises a flexible material

4. The primary container assembly of any one of the preceding claims, wherein the distal end (24b) comprises a Luer connection integrally attached to the first end (64a) of the tube (60).

5. The primary container assembly of any one of the preceding claims, further comprising:
a needle hub (80) defining an interface between the hollow needle (100) and the tube (60).

6. The primary container assembly of claim 5, further comprising:
a needle shield (110) disposed over at least a portion of the hollow needle (100) and releasably connected to the needle hub (80) so as to maintain the hollow needle (100) in a sterile condition.

7. The primary container assembly of claim 6, wherein the needle shield (110) is frictionally connected to the needle hub (80).

8. The primary container assembly of claim 1, wherein the cup (130) is releasably attached to the body (24).

9. The primary container assembly of claim 7, wherein the needle shield (110) is releasably secured to the cup (130).

10. The primary container assembly of claim 9, wherein when the needle shield (110) is detached from the cup (130), the tube (60) is configured to be transitioned from the coiled configuration to an uncoiled configuration, in which the tube (60) is uncoiled from around the portion of the distal end (24b).

11. The primary container assembly of any one of claims 6, 7, 9, and 10, wherein the cup (130) is a first cup (130),
wherein the primary container assembly further comprising a second cup (160) releasably attached to the first cup (130),
wherein the needle shield (110) is substantially received within the second cup (160) such that the tube (60) maintains a concentric shape, and
wherein the second cup (160) is configured to interface with a filling machine.

12. The primary container assembly of any one of the preceding claims, wherein the hollow needle (100) is a first needle (100), and
wherein the primary container assembly further comprises a second needle (72) defining an interface between the distal end (24b) and the first end (64a) of the tube (60).

13. The primary container assembly of any one of the preceding claims, wherein the tube (60) defines a first length measured along an axis coaxial with the body (24) of the tube (60) from the first end (64a) to the second end (64b), and the hollow needle (100) defines a second length, wherein the first length is greater than the second length.

14. A drug dispensing device (200), comprising:
the primary container assembly according to any one of the preceding claims, wherein the primary container assembly further comprises a needle hub (80) defining an interface between the hollow needle (100) and the tube (60); and
an input (208) configured to engage the needle hub (80) such that the tube (60) is in fluid communication with the input (208).

## Patentansprüche

1. Primärbehälteranordnung, umfassend:
einen Körper (24), aufweisend:
ein proximales Ende (24a), das eine Öffnung (28) definiert, die dazu ausgelegt ist, einen Kolben (50) aufzunehmen;
ein distales Ende (24b) gegenüber dem proximalen Ende (24a) und einen Auslass (32) definierend; und
eine Kammer (26), die sich von dem proximalen Ende (24a) zu dem distalen Ende (24b) erstreckt, die dazu ausgelegt ist, ein Medikament aufzunehmen;
eine Röhre (60), die sich von einem ersten Ende (64a), das einstückig an dem distalen Ende (24b) angebracht ist, zu einem zweiten Ende (64b) gegenüber dem ersten Ende (64a) erstreckt, wobei die Röhre (60) einen Kanal (68) definiert, der sich von dem ersten Ende (64a) zu dem zweiten Ende (64b) erstreckt;
eine Hohlnadel (100), die dazu ausgelegt ist, die Haut eines Patienten zu durchstechen, wobei die Hohlnadel (100) einstückig an dem zweiten Ende (64b) der Röhre (60) angebracht ist; und
wobei die Röhre (60) dazu ausgelegt ist, das Medikament von der Kammer (26) zu der Hohlnadel (100) zu lenken, **dadurch gekennzeichnet, dass** die Primärbehälteranordnung ferner einen Aufsatz (130) umfasst, der an dem distalen Ende (24b) angebracht ist und
wobei die Röhre (60) dazu ausgelegt ist, in eine gewundene Auslegung platziert zu werden, bei der ein Abschnitt der Röhre (60) um einen Abschnitt des distalen Endes (24b) gewickelt und im Wesentlichen in dem Aufsatz (130) angeordnet ist.

2. Primärbehälteranordnung nach Anspruch 1, umfassend einen Primärbehälter, der den Körper (24) definiert.

3. Primärbehälteranordnung nach Anspruch 1 oder Anspruch 2, wobei die Röhre (60) ein flexibles Material umfasst.

4. Primärbehälteranordnung nach einem der vorhergehenden Ansprüche, wobei das distale Ende (24b) eine Luer-Verbindung umfasst, die einstückig an dem ersten Ende (64a) der Röhre (60) angebracht ist.

5. Primärbehälteranordnung nach einem der vorhergehenden Ansprüche, ferner umfassend:
einen Nadelansatz (80), der eine Schnittstelle zwischen der Hohlnadel (100) und der Röhre (60) definiert.

6. Primärbehälteranordnung nach Anspruch 5, ferner umfassend:
einen Nadelschutz (110), der über mindestens einem Abschnitt der Hohlnadel (100) angeordnet und lösbar mit dem Nadelansatz (80) verbunden ist, um die Hohlnadel (100) in einem sterilen Zustand zu halten.

7. Primärbehälteranordnung nach Anspruch 6, wobei der Nadelschutz (110) kraftschlüssig mit dem Nadelansatz (80) verbunden ist.

8. Primärbehälteranordnung nach Anspruch 1, wobei der Aufsatz (130) lösbar an dem Körper (24) angebracht ist.

9. Primärbehälteranordnung nach Anspruch 7, wobei der Nadelschutz (110) kraftschlüssig an dem Aufsatz (130) befestigt ist.

10. Primärbehälteranordnung nach Anspruch 9, wobei, wenn der Nadelschutz (110) von dem Aufsatz (130) gelöst wird, die Röhre (60) dazu ausgelegt ist, aus der gewundenen Auslegung in eine abgewickelte Auslegung überführt zu werden, in der die Röhre (60) von dem Abschnitt des distalen Endes (24b) abgewickelt ist.

11. Primärbehälteranordnung nach einem der Ansprüche 6, 7, 9 und 10, wobei der Aufsatz (130) ein erster Aufsatz (130) ist,
wobei die Primärbehälteranordnung ferner einen zweiten Aufsatz (160) umfasst, der lösbar an dem ersten Aufsatz (130) angebracht ist,
wobei der Nadelschutz (110) im Wesentlichen in dem zweiten Aufsatz (160) aufgenommen ist, so dass die Röhre (60) eine konzentrische Form behält, und
wobei der zweite Aufsatz (160) dazu ausgelegt ist, eine Schnittstelle mit einer Füllmaschine zu bilden.

12. Primärbehälteranordnung nach einem der vorhergehenden Ansprüche, wobei die Hohlnadel (100) eine erste Nadel (100) ist, und
wobei die Primärbehälteranordnung ferner eine zweite Nadel (72) umfasst, die eine Schnittstelle zwischen dem distalen Ende (24b) und dem ersten Ende (64a) der Röhre (60) definiert.

13. Primärbehälteranordnung nach einem der vorhergehenden Ansprüche, wobei die Röhre (60) eine erste Länge definiert, gemessen entlang einer Achse koaxial zu dem Körper (24) der Röhre (60) von dem ersten Ende (64a) zu dem zweiten Ende (64b), und die Hohlnadel (100) eine zweite Länge definiert, wobei die erste Länge größer ist als die zweite Länge.

14. Medikamentenabgabevorrichtung (200), umfassend:
die Primärbehälteranordnung nach einem der vorhergehenden Ansprüche, wobei die Primärbehälteranordnung ferner einen Nadelansatz (80) umfasst, der eine Schnittstelle zwischen der Hohlnadel (100) und der Röhre (60) definiert; und
einen Eingang (208), der dazu ausgelegt ist, den Nadelansatz (80) derart in Eingriff zu nehmen, dass die Röhre (60) in Fluidverbindung mit der Eingabe (208) ist.

## Revendications

1. Ensemble récipient primaire, comprenant :
un corps (24) ayant :
une extrémité proximale (24a) définissant une ouverture (28) configurée pour recevoir un piston (50) ;
une extrémité distale (24b) opposée à l'extrémité proximale (24a) et définissant une sortie (32) ; et
une chambre (26) s'étendant de l'extrémité proximale (24a) à l'extrémité distale (24b) qui est configurée pour recevoir un médicament ;
un tube (60) s'étendant d'une première extrémité (64a), qui est fixée de manière intégrée à l'extrémité distale (24b), à une seconde extrémité (64b) opposée à la première extrémité (64a), le tube (60) définissant un canal (68) qui s'étend de la première extrémité (64a) à la seconde extrémité (64b) ;
une aiguille creuse (100) configurée pour pénétrer dans la peau d'un patient, l'aiguille creuse (100) étant fixée de manière intégrée à la seconde extrémité (64b) du tube (60) ; et
le tube (60) étant configuré pour diriger le médicament de la chambre (26) à l'aiguille creuse (100),
**caractérisé en ce que** l'ensemble récipient primaire comprend en outre une coupelle (130) fixée à l'extrémité distale (24b) et
le tube (60) étant configuré pour être placé dans une configuration enroulée, dans laquelle une partie du tube (60) est enroulée autour d'une partie de l'extrémité distale (24b) et sensiblement disposée à l'intérieur de la coupelle (130).

2. Ensemble récipient primaire selon la revendication 1, comprenant un récipient primaire qui définit le corps (24).

3. Ensemble récipient primaire selon la revendication 1 ou selon la revendication 2, le tube (60) comprenant un matériau flexible.

4. Ensemble récipient primaire selon l'une quelconque des revendications précédentes, l'extrémité distale (24b) comprenant un raccord Luer fixé de manière intégrée à la première extrémité (64a) du tube (60).

5. Ensemble récipient primaire selon l'une quelconque des revendications précédentes, comprenant en outre :
un moyeu d'aiguille (80) définissant une interface entre l'aiguille creuse (100) et le tube (60).

6. Ensemble récipient primaire selon la revendication 5, comprenant en outre :
une protection d'aiguille (110) disposée sur au moins une partie de l'aiguille creuse (100) et raccordée de manière amovible au moyeu d'aiguille (80) de manière à maintenir l'aiguille creuse (100) dans un état stérile.

7. Ensemble récipient primaire selon la revendication 6, la protection d'aiguille (110) étant raccordée par friction au moyeu d'aiguille (80).

8. Ensemble récipient primaire selon la revendication 1, la coupelle (130) étant fixée de manière amovible au corps (24).

9. Ensemble récipient primaire selon la revendication 7, la protection d'aiguille (110) étant fixée de manière amovible à la coupelle (130).

10. Ensemble récipient primaire selon la revendication 9, lorsque la protection d'aiguille (110) est détachée de la coupelle (130), le tube (60) étant configuré pour passer de la configuration enroulée à une configuration non enroulée, dans laquelle le tube (60) est déroulé d'autour de la partie de l'extrémité distale (24b).

11. Ensemble récipient primaire selon l'une quelconque des revendications 6, 7, 9 et 10, la coupelle (130) étant une première coupelle (130),
l'ensemble récipient primaire comprenant en outre une seconde coupelle (160) fixée de manière amovible à la première coupelle (130),
la protection d'aiguille (110) étant sensiblement reçue à l'intérieur de la seconde coupelle (160) de sorte que le tube (60) conserve une forme concentrique, et
la seconde coupelle (160) étant configurée pour s'interfacer avec une machine de remplissage.

12. Ensemble récipient primaire selon l'une quelconque des revendications précédentes, l'aiguille creuse (100) étant une première aiguille (100), et
l'ensemble récipient primaire comprenant en outre une seconde aiguille (72) définissant une interface entre l'extrémité distale (24b) et la première extrémité (64a) du tube (60).

13. Ensemble récipient primaire selon l'une quelconque des revendications précédentes, le tube (60) définissant une première longueur mesurée le long d'un axe coaxial avec le corps (24) du tube (60) de la première extrémité (64a) à la seconde extrémité (64b), et l'aiguille creuse (100) définissant une seconde longueur, la première longueur étant supérieure à la seconde longueur.

14. Dispositif de distribution de médicaments (200), comprenant :
l'ensemble récipient primaire selon l'une quelconque des revendications précédentes, l'ensemble récipient primaire comprenant en outre un moyeu d'aiguille (80) définissant une interface entre l'aiguille creuse (100) et le tube (60) ; et
une entrée (208) configurée pour venir en prise avec le moyeu d'aiguille (80) de sorte que le tube (60) soit en communication fluide avec l'entrée (208).
